(19) Europäisches Patentamt / European Patent Office / Office européen des brevets

(11) **EP 3 509 566 B1**

(12) **EUROPEAN PATENT SPECIFICATION**

(45) Date of publication and mention of the grant of the patent:
**04.11.2020 Bulletin 2020/45**

(21) Application number: **17758089.1**

(22) Date of filing: **14.08.2017**

(51) Int Cl.:
***A61K 8/49*** *(2006.01)* ***A61Q 19/02*** *(2006.01)*

(86) International application number:
**PCT/EP2017/070550**

(87) International publication number:
**WO 2018/046243 (15.03.2018 Gazette 2018/11)**

(54) **COMPOUNDS FOR REDUCING CELLULAR MELANIN CONTENT**

VERBINDUNGEN ZUR VERRINGERUNG DES ZELLULÄREN MELNINGEHALTS

COMPOSÉS POUR LA REDUCTION DU CONTENU CELLULAIRE DE MELANIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.09.2016 EP 16187446**

(43) Date of publication of application:
**17.07.2019 Bulletin 2019/29**

(73) Proprietors:
- **Unilever N.V.**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**
- **Unilever PLC**
  **London, Greater London EC4Y 0DY (GB)**
  Designated Contracting States:
  **CY GB IE MT**

(72) Inventors:
- **STOTT, Ian, Peter**
  **Wirral Merseyside CH63 3JW (GB)**
- **CHANDRAMOWLI, Ganesh**
  **Bangalore 560066 (IN)**
- **THIMMAIAH, Sreenivasa**
  **Bangalore 560066 (IN)**

(74) Representative: **James, Helen Sarah**
**Unilever Patent Group**
**Colworth House**
**Sharnbrook**
**Bedford MK44 1LQ (GB)**

(56) References cited:
**EP-A1- 2 196 190**
**EP-A1- 2 412 701**
**EP-A2- 0 820 767**
**WO-A1-98/06695**
**WO-A1-2016/107697**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 509 566 B1

## Description

### Field of the invention

The present invention relates to molecules capable of reducing cellular melanin content of human skin; in particular to reducing melanin content for applications in fields such as skin lightening.

### Background of the invention

Most people are concerned with hyper pigmented skin. For example, people with age spots or freckles may wish such pigmented spots to be less pronounced, while others may wish to reduce the skin darkening caused by exposure to sunlight. Some others may wish to lighten their natural skin colour or tone. To meet this need, attempts were made to develop products that regulate the pigment production in melanocytes. However, the substances identified thus far tend to have either lower efficacy or undesirable side effects, such as, for example, toxicity or skin irritation. Therefore, there is a continuing need for new skin lightening agents, with improved overall effectiveness.

Desired skin colour is a major unmet consumer need around the world and especially in Asia. Consumers particularly desire an even skin colour as opposed to uneven tone, absence of age spots (solar lentigines), absence of hyperpigmentation and lighter overall skin tone. One solution is to use biological actives that reduce the activity of melanocytes. These cells, present in the basal layer of the epidermis make the dark coloured pigment melanin and export it, in small export vesicles called melanosomes, to the neighbouring keratinocytes. It is described that compounds which reduce melanin synthesis when topically applied to the skin will reduce skin darkness over time and can generate a more even skin tone. Tyrosinase is a popular target for the regulation of melanocyte production. However, some inhibitors of tyrosinase are associated with safety issues, for example, melanocyte cell death, permanent depigmentation, irritation and allergic reactions. Often, effective inhibitors kill melanocytes (for example hydroquinone) or cause sensitisation. There is therefore a need for safe and effective inhibitors of skin pigment production that work through an alternative and safe mechanism.

Thanigaimalai et al (Bioorganic & Medicinal Chem. Letters, 21, 6824-6828 (2011)) describes a study of the effect of a series of 1 -phenylthioureas and 1 ,3-disubstituted thioureas against melanin formation in melanoma B16 cell line and mushroom tyrosinase. Inhibitory activity of tyrosinase of the 1 -phenylthioureas is parallel to their melanogenic inhibition. Thus, the melanogenic inhibition in melanoma B16 cells of the 1 -phenylthioureas could be the result of inhibition of tyrosinase. However the 1 ,3-disubstituted thioureas appear as melanogenic inhibitors without inhibition of tyrosinase.

CN 19 03 203 (Fudan University) discloses use of a polysubstituted acyl thiouracil derivative in preparing an anti-viral medicine.

US 2006/0135618 (Jean et al) discloses a medicine or a cosmetic composition comprising at least one generic thiourea, or at least one of its monoxide or dioxide derivatives, or mixtures thereof. The medicine is advantageously used for inhibiting tyrosinase, and as an anti-mutagenic and anti-carcinogenic agent.

US 2002/0044914 (Dooley et al) discloses methods and formulations for reducing pigmentation of skin using an array of compounds selected from benimidazoles, phenylthioureas, phenylthiols, phenylamines, bi- and multicyclic phenols, thiopheneamines and benzothiamides.

JP 56 32460 (Nihon Tokushu Noyaku Seizo) discloses acyl pyridyl thiourea derivatives of generic formula which are useful in controlling certain pathogenic fungi.

Liu et al (Chem. Res. Chinese Universities, 26(6), 929-932 (2010)) discloses synthesis of a series of compounds derived from 2,4- dichlorophenoxyacetyl(thio)urea and S-(+)-3-methyl-2-(4-chlorophenyl) butyrannide and their subsequent testing for herbicidal and fungicidal activity.

EP2414701 A1(Ajinonoto, 2012) discloses certain indole compounds having a melanin production suppressive activity.

The indolyl compounds have the following formula:

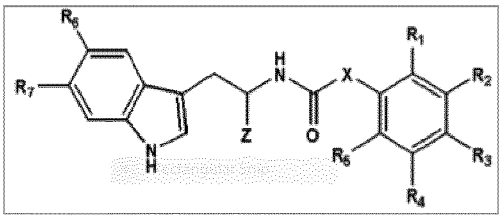

WO9806695 A1 (Sederma) discloses the synthesis and use of lipophilic melatonin homologues of the following general formula in which R = a C11 to C19 alkyl chain, linear or branched, saturated or unsaturate, hydroxylated or not. These derivatives are preferably obtained by 5-methoxy-tryptemin acylation. They are designed for use in cosmetic or dermopharmaceutical compositions for hydrating, regenerating, anti-seborrheic, anti-wrinkle, bleaching skin treatment and for the prevention of actinic damage caused by the sun and by the atmosphere.

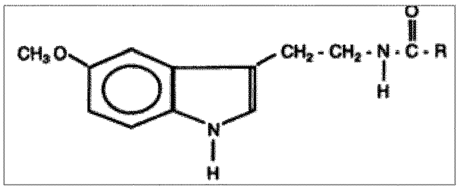

EP0820767 A2 (L'Oreal, 1998) discloses dermocosmetic compositions for whitening/depigmenting human skin, comprise a skin-whitening/depigmenting effective amount of at least one melatonin derivative having the following structural formula: in which R1 is a lower alkyl radical, R2 is a hydrogen atom or a lower alkyl radical, and R3 is a hydrogen atom or a lower acyl radical, with the proviso that the hydroxyl radical is in the 4-, 6- or 7-position on the indole ring system, or a physiologically acceptable salt, solvate or bioprecursor/prodrug thereof, formulated into a topically applicable, cosmetically/dermatologically acceptable vehicle, carrier or diluent therefor.

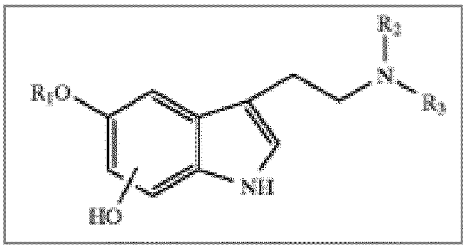

EP2196190 A1 (Cognis, 2010) discloses methods for reducing pigmentation or hyperpigmentation of skin comprising applying to the skin a substance of formula (I) in the D-form, L-form or racemic form (D,L) are disclosed: wherein R1 is selected from the group consisting of a hydrogen group (-H), a methyl group (-CH3), an ethyl group (-CH2CH3), a C3 alkyl group, a C4 alkyl group, an acetyl group (-C(O)CH3), a propanoyl group (-C(O)CH2CH3), an n-butanoyl group (-C(O)CH2CH2CH3), and a 2-methyl-propanoyl group (-C(O)CH(CH3)2), R2 is selected from the group consisting of a hydrogen group (-H), an ethyl group (CH2CH3), a C3 alkyl group, a C4 alkyl group, an acetyl group (-C(O)CH3), a propanoyl group (-C(O)CH2CH3), an n-butanoyl group (-C(O)CH2CH2CH3), and a 2-methyl-propanoyl group (-C(O)CH(CH3)2), R3 is selected from the group consisting of a hydroxyl group (-OH), an amino group (NH2), a methoxy group (OCH3), an ethoxy group (-OCH2CH3), a C3 alkoxy group, a C4 alkoxy group, and a benzyloxy group (-OCH2C6H5), and X and Y are independently selected from the group consisting of a hydrogen group (-H), a methyl group (-CH3), an ethyl group (-CH2CH3), a C3 group alkyl, a C4 alkyl group, a halogen group, a methoxy group (-OCH3), an ethoxy group (-OCH2CH3), a C3 alkoxy group, a C4 alkoxy group, and a benzyloxy group (-OCH2C6H5), provided that at least one of X and Y is not a hydrogen group (-H). Cosmetic compositions and/or topical compositions comprising the substance are useful for (i) lightening of the skin and/or (ii) whitening of the skin and/or (iii) reduction of pigmentation of the skin and/or (iv) reduction of hyperpigmentation of the skin and/or (v) inhibition of melanogenesis in the skin and/or (vi) prevention and/or retardation of signs of ageing and/or improving the skin appearance of an aged skin. Such composition are also useful for treatment of pigmentation or hyperpigmentation of the skin due to abnormal melanin synthesis and/or secretion.

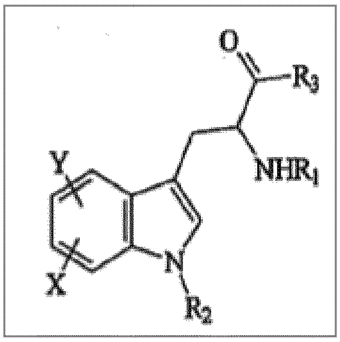

Conventional skin lightening compositions are based on the use of skin lightening agents that are believed to control dispersion of melanin or to inhibit tyrosinase. These skin- lightening agents include niacinamide, carboxylic acids like azelaic acid and kojic acid, plant extracts and hydroquinone. Indole type of compounds have earlier been claimed for cosmetic benefits. Further, indolyl compounds, which contain a core indole group carrying other chemical groups are also known. Such compounds are commercially available but not known for reducing melanin content of human skin.
Further compounds having melanin suppressive activity are disclosed in EP 2412701.
It is therefore an object of the present invention to provide a compound that reduces melanin content.
Surprisingly, it is found that specific indolyl compounds can reduce the cellular melanin content of melanocytes in human skin. The inventors have observed that selected compounds of the same generic structure are able to effectively reduce melanin in human cell living skin equivalents containing keratinocytes and melanocytes (so called Melanoderma™). Melanoderma™ are in vitro 3D living skin equivalents containing both human keratinocytes and melanocytes and are used as a close mimic of the effect of compounds on human skin function in vivo.
Accordingly, the aforementioned generic structure describes compounds which inhibit pigment production and which can be used to beneficially alter skin colour and treat hyperpigmentation disorders in a safe and effective manner.

**Summary of the invention**

In accordance with a first aspect is disclosed the use of a compound of formula (I) or a salt thereof, for reducing cellular melanin content of human skin, where,

(i) R is:

(a) - $(CH_2)_n$ - A, where n = 1 to 6 and A is -OH or -NH2; or
(b) - O-$(CH_2)_n$ - X, where n = 1 to 5 and X is a halogen;

(ii) $R_2$ is -H, alkyl, n-hexyl, aryl, alkoxy or aryloxy group; and,
(iii) $R_3$ is -H, alkyl, aryl, alkoxy or aryloxy group.

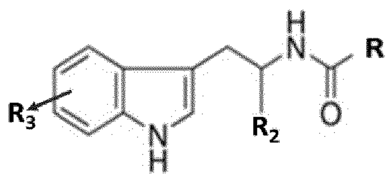

(I)

wherein said use is for cosmetic purpose.
These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of

materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

## Detailed description of the invention

"Personal Care Composition" as used herein, is meant to include a composition for topical application to sun-exposed areas of the skin and/or hair of humans. Such a composition may be classified as leave-on or rinse off, and includes any product applied to a human body for improving appearance, cleansing, odour control or general aesthetics. The compositions of the present invention can be in the form of a liquid, lotion, cream, foam, scrub, gel, or toner, or applied with an implement or via a facemask, pad or patch. Non-limiting examples of such compositions include leave-on skin lotions, creams, antiperspirants, deodorants, foundations, mascara, sunscreen lotions and wash-off shampoos, conditioners and shower gels. The compositions of the present invention are preferably a leave-on composition.

"Skin" as used herein is meant to include skin on the face and body (e.g., neck, chest, back, arms, underarms, hands, legs and scalp) and especially to the sun exposed parts thereof. Compositions are also of relevance to applications on any other keratinous substrates of the human body e.g. hair where products may be formulated with the specific aim of improving photoprotection.

In accordance with a first aspect is disclosed the use of a compound of formula (I) or a salt thereof, for reducing cellular melanin content of human skin, where,

(i) R is:

(a) - $(CH_2)_n$ - A, where n = 1 to 6 and A is -OH or -NH2; or
(b) - O-$(CH_2)_n$ - X, where n = 1 to 5 and X is a halogen;

(ii) $R_2$ is -H, alkyl, n-hexyl, aryl, alkoxy or aryloxy group; and,
(iii) $R_3$ is -H, alkyl, aryl, alkoxy or aryloxy group.

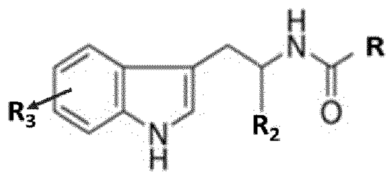

(I)

wherein said use is for cosmetic purpose.

In one aspect it is preferred that when R is - O-$(CH_2)_n$ - X, the n = 2 and the X is chlorine, bromine or fluorine. Alternatively when the R is -$(CH_2)_n$ - A, the n = 1 and the A is -OH or -$NH_2$.

Further preferably the R is -O-$CH_2$-$CH_2$-Cl or -$CH_2$ - $NH_2$

It is preferred that $R_2$ is a alkyl group. More preferably, $R_2$ is methyl group.

It is preferred that $R_3$ is -H.

It is preferred that the salt of the compound of the formula (I) is an acetate.

It is particularly preferred that the compound of the formula (I) is 2-chloroethyl [2-(1*H*-indol-3-yl)-1-methylethyl] carbamate.

Alternatively it is preferred that the compound of the formula (I) is 2-amino-N-[2-(1H-indol-3-yl)-1-methylethyl] acetamide acetate.

The compounds of the formula (I) are useful in applications of skin lightening. The benefits inter alia may include treatment of melasma, treating post-inflammatory hyperpigmentation, treating age spots, treating dark spots and treating uneven skin tone.

It is preferred that melanin is contained within human primary melanocytes.

The compounds of the formula (I) can be used in compositions for cosmetic benefits. These benefits are inter alia for care and preservation of healthy skin and in that sense, such benefits are not the same as medicinal or therapeutic benefits.

Accordingly, in one aspect the use of a compound of formula (I) or a salt thereof, for reducing cellular melanin content of human skin is for cosmetic purpose.

Alternatively, in another aspect the use of a compound of formula (I) or a salt thereof, for reducing cellular

melanin content of human skin is for non-cosmetic purpose which includes therapeutic or pharmaceutical purpose. Such purpose includes treatment of any medical condition which results in increased or abnormal melanin content in human skin.

Method and other aspects

In accordance with a second aspect is disclosed a method of reducing cellular melanin content of human skin comprising a step of applying thereto a compound of the formula (I) or a salt thereof, as disclosed in the first aspect.
The method is a cosmetic method.
In accordance with a third aspect is disclosed a method of reducing cellular melanin content of human skin comprising a step of applying to a person in need thereof, a compound of the formula (I) or a salt thereof, as disclosed in the first aspect.
The method is a cosmetic method.
The composition is intended primarily as a product for topical application to human skin, especially as an agent for protection from solar radiation, and preventing or reducing the appearance of wrinkled or aged skin or age spots or any other sign of increased or impaired melanin content in the skin.
In use, a small quantity of the composition, for example from 1 to 5 ml, is applied to exposed area of the skin, from a suitable container or applicator and, if necessary, it is then spread over and/or rubbed into the skin using the hand or fingers or a suitable device.
In accordance with a fifth aspect is disclosed a compound of the formula (I) or a salt thereof as disclosed in the first aspect for use to reduce cellular melanin content of human skin.

Skin Lightening Compositions

The invention can be made industrially applicable through skin lightening compositions. Such compositions contain other ingredients which are described hereinafter.

Skin Benefit Agents

Such a composition may additionally include a skin benefit agent selected from the group comprising hydroxy acids, polyhydroxy acids, hydroxy fatty acids in particular 12-hydroxy stearic acid, kojic acid, depigmenting oligopeptides, galardin, polyphenol antioxidants, thiolic antioxidants, cysteamine hydrochloride, hydroquinone, t-butyl hydroquinone, vitamin C derivatives, vitamin E derivatives, vitamin B derivatives, retinoids, 4-substituted resorcinol derivatives, and mixtures thereof.

Cosmetically Acceptable Vehicle

The skin lightening composition may further comprise a cosmetically acceptable vehicle which may act as diluents, dispersants and/or carriers for the skin lightening agents used in the composition, to facilitate their distribution when the composition is applied to the skin. The cosmetically acceptable vehicle suitable for use in skin lightening compositions may be aqueous, anhydrous or an emulsion; aqueous or an emulsion, especially water-in-oil or oil-in-water emulsion being most preferred. Water, when present, typically makes up the balance of the composition.
Besides water, organic solvents may also serve as carriers.

Emollients

Emollients may also be used as cosmetically acceptable carriers in skin lightening compositions. Emollients are generally in the form of silicone oils and synthetic esters. Silicone oils may be volatile or non-volatile. Volatile silicone oils are preferably chosen from cyclic or linear polydimethylsiloxanes containing from 3 to 9, preferably from 4 to 5, silicon atoms. Non-volatile silicone oils useful as emollients include polyalkyl siloxanes, polyalkylaryl siloxanes and polyether siloxane copolymers. The essentially non-volatile polyalkyl siloxanes useful herein include, for example, polydimethyl siloxanes.
Ester emollients that may be used are:

(i) alkenyl or alkyl esters of fatty acids having 10 to 20 carbon atoms. Examples thereof include isoarachidyl neopentanoate, isononyl isonanonoate, oleyl myristate, oleyl stearate, and oleyl oleate;
(ii) ether-esters such as fatty acid esters of ethoxylated fatty alcohols;
(iii) polyhydric alcohol esters such as ethylene glycol mono- and di-fatty acid esters, diethylene glycol mono- and

di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters;

(iv) wax esters such as beeswax, spermaceti, myristyl myristate, stearyl stearate and arachidyl behenate; and

(v) sterols esters, of which cholesterol fatty acid esters are examples.

Emollients may be present in the composition anywhere from 0.1 to 50 %, preferably from 1 to 20 % by weight of the composition.

Fatty Acids

Fatty acids having from 10 to 30 carbon atoms may also be included as cosmetically acceptable carriers. Illustrative examples of such fatty acids are pelargonic, lauric, myristic, palmitic, stearic, isostearic, hydroxystearic, oleic, linoleic, ricinoleic, arachidic, behenic, erucic acids and mixtures thereof.

Humectants

Humectants of the polyhydric alcohol type may also be employed as cosmetically acceptable carriers. Humectants aid in increasing the effectiveness of the emollient, reduces scaling, stimulates removal of built-up scale and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably propylene glycol or sodium hyaluronate. The concentration of humectant in the composition may range anywhere from 0.5 to 30 %, preferably between 1 and 15 % by weight of the composition.

Thickeners

Thickeners may also be utilized as part of the cosmetically acceptable carrier of the compositions. Typical thickeners include crosslinked acrylates (e.g. Carbopol 982), hydrophobically-modfied acrylates (e.g. Carbopol 1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitably include guar, xanthan, sclerotium, carrageenan, pectin and combinations of these gums. Concentration of the thickener in the composition may range from 0.0001 to 5 %, usually from 0.001 to 1 %, optimally from 0.01 to 0.5 % by weight of the composition. Collectively the water, solvents, silicones, esters, fatty acids, humectants and/or thickeners will constitute the cosmetically acceptable carrier in amounts from 1 to 99.9 %, preferably from 80 to 99 % by weight of the composition.

Surfactants

Surfactants may also be present in the composition. Total concentration of the surfactant will range from 0.1 to 40 %, preferably from 1 to 20 %, optimally from 1 to 5 % by weight of the composition. The surfactant may be selected from the group consisting of anionic, nonionic, cationic and amphoteric actives. Particularly preferred nonionic surfactants are those with a $C_{10}$-$C_{20}$ fatty alcohol or acid hydrophobe condensed with from 2 to 100 moles of ethylene oxide or propylene oxide per mole of hydrophobe; $C_2$-$C_{10}$ alkyl phenols condensed with from 2 to 20 moles of alkylene oxide; mono- and di- fatty acid esters of ethylene glycol; fatty acid monoglyceride; sorbitan, mono- and di- $C_8$-$C_{20}$ fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfate and sulfonates, alkyl sulfates and sulfonates, alkylbenzene sulfonates, alkyl and dialkyl sulfosuccinates, $C_8$-$C_{20}$ acyl isethionates, acyl glutamates, $C_8$-$C_{20}$ alkyl ether phosphates and combinations thereof.

Sunscreens

Sunscreens include those materials commonly employed to block ultraviolet light. Illustrative compounds are

the derivatives of PABA, cinnamate and salicylate. For example, avobenzophenone (Parsol 1789®) octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively. Ecamsule, a benzylidene camphor derivative, sold under the trade mark Mexoryl SX, and drometrizole trisiloxane, a benzotriazole sold under the trade mark Mexoryl XL, may also be used. Still other examples include octocrylene, phenylbenzimidazole sulfonic acid (also known as ensulizole), ethylhexyl salicylate,diethylhexyl naphthylate, bimotrizinole (trade marked as Tinosorb S) and bisoctrizole (Tinosorb M). Inorganic sunscreens include oxides like titanium dioxide and zinc oxide which reflect or scatter the suns rays. The exact amount of sunscreen employed in the compositions can vary depending upon the degree of protection desired from the sun's UV radiation. Additives that reflect or scatter the sun rays may also be employed. These additives include oxides like zinc oxide and titanium dioxide.

Fragrances

Fragrances may be used in the topical skin lightening composition. Illustrative non-limiting examples of the types of fragrances that may be used include those comprising terpenes and terpene derivatives like those described in Bauer, K., et al., Common Fragrance and Flavor Materials, VCH Publishers (1990). Illustrative yet non-limiting examples of the types of fragrances that may be used in this invention include myrcene, dihydromyrenol, citral, tagetone, cis-geranic acid, citronellic acid, mixtures thereof or the like.

The CTFA Cosmetic Ingredient Handbook, Second Edition, 1992, which is incorporated by reference herein in its entirety, describes a wide variety of non-limiting cosmetic and pharmaceutical ingredients commonly used in the skin care industry.

Various optional active ingredients may be used in the topical skin lightening compositions. Actives are defined as skin benefit agents other than emollients and other than ingredients that merely improve the physical characteristics of the composition. Although not limited to this category, general examples include extender pigments such as talcs and silicas, as well as alpha- hydroxy acids, beta-hydroxy acids and zinc salts.

Beta-hydroxy acids include salicylic acid, for example. Zinc oxide and zinc pyrithione are examples of zinc salts useful in the topical skin lightening composition.

Many compositions, especially those containing water, should be protected against the growth of potentially harmful microorganisms. Anti-microbial compounds, such as triclosan, and preservatives are, therefore, typically necessary. Suitable preservatives include alkyl esters of p-hydroxybenzoic acid, hydantoin derivatives, propionate salts, and a variety of quaternary ammonium compounds. Particularly preferred preservatives are methyl paraben, propyl paraben, phenoxyethanol and benzyl alcohol. Preservatives will usually be employed in amounts ranging from 0.1 to 2 % by weight of the compositions.

Still other optional ingredients that may be used with composition include dioic acids (e.g. malonic acid and sebacic acid), antioxidants like vitamin E, retinoids, including retinoic acid, retinal, retinol and retinyl esters such as retinyl propionate and retinyl palmitate, conjugated linoleic acid, petroselinic acid and mixtures thereof, as well as any other conventional ingredients well known for wrinkle-reducing (such as hyaluronic acid, ubiquinone, jasmonic acid derivatives, collagen, peptides and proxylane), anti-acne effects and reducing the impact of sebum.

The packaging for the topical skin lightening composition can be a patch, bottle, tube, roll-ball applicator, propellant driven aerosol device, squeeze container or lidded jar.

The invention will now be further illustrated by means of the following non-limiting examples.

**Examples**

A description of the concerned experimental procedure is as follows.

A set of indolyl compounds were purchased from SIGMA-ALDRICH as stocks of 10 mM in 100% DMSO. They were initially examined at 25 uM concentration in cell culture (Table 1) for their ability to reduce melanin content and then, at different doses (Table 2). Neonatal foreskin primary human epidermal melanocytes was sourced from Cascade Biologicals (labelled passage $P_0$). Melanocytes were maintained in Medium 254CF (Cascade Cat. # M-254CF-500) supplemented with human melanocyte growth supplement (Cascade; Cat. # S-002-5), hereafter referred to as MGM. Cells were maintained at 37 °C in a humidified incubator with 5 % $CO_2$ atmosphere.

Cell viability and melanin content assay

50,000 cells were seeded in 24 well plate in MGM. After 24 hours, the cultures were treated with various concentrations of test materials and were left undisturbed for a further period of 72 hours. Comparative vehicle controls of 0.25 % (v/v) DMSO were also set up in parallel, simultaneously. At the end of the incubation period, the cell viability

was determined using the calcein method.

Briefly, cell culture spent media was removed and cells washed once with 0.4 ml of 1x PBS-Ca-Mg solution. Fresh 1 μM calcein-AM was added (0.2 ml/well), including to the control wells without cells. Plates were covered with aluminium foil and incubated for 30 minutes at 37 °C in the regular $CO_2$ incubator. Calcein fluorescence was then measured ($\lambda_{ex}$ 490 nm and $\lambda_{em}$ 520 nm) in TECAN M1000 Infinite series plate reader.

Melanin Content Assay

After the calcein fluorescence readings were obtained the cells were drained and added fresh 125 μl of 1N NaOH (in 10% DMSO) per well. Cells were lysed by resuspension and incubation (60 °C/one hour.). Then this lysate was transferred to a fresh 384-well plate and measured $OD_{405nm}$ in a TECAN M1000 plate reader (estimate of relative melanin content).

Calculations

Calcein fluorescence values were ratio converted in 0 to 100 scale (% Viability), with 100 representing the value of the 0.25% DMSO sample. Percentage Melanin content was calculated as the ratio between $OD_{405nm}$ value of any sample to that of DMSO reference sample. Normalized melanin content value was then calculated as 100*(% Melanin Content)/(% Cellular viability).

% Reduction in melanin content was estimated as [100-(Normalized melanin content)]

Example 1: Technical effect of compounds on melanin content

The following three compounds were tested. Compounds 1 and 3 were within the scope of the invention. Compound 2, although having a similar structure, was outside the scope of the invention.

Compound #1: 2-chloroethyl [2-(1H-indol-3-yl)-1-methylethyl] carbamate

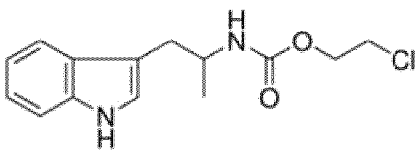

Compound #2: N-[2-(1H-indol-3-yl)-1-methylethyl]acetamide

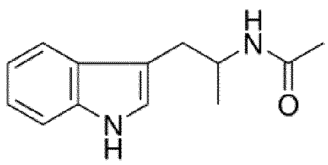

Compound #3: 2-amino-N-[2-(1H-indol-3-yl)-1-methylethyl]acetamide acetate

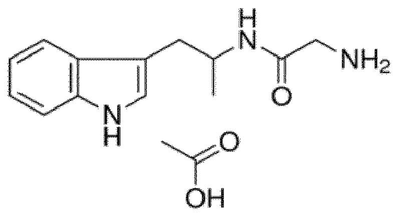

Out of all the indolyl compounds tested, compound #1 and 3 showed significant melanin reduction.

Table 1: Cellular Melanin Content Data

| Material Tested | Calcein Fluorescence (arbitrary unit) | % Viability | $OD_{405}$nm (Melanin) | % Melanin | 100* (%Melanin/% Viability) | % reduction in Melanin |
|---|---|---|---|---|---|---|
| 0.25% v/v DMSO control | 100 | 100 | 0.112 | 100 | 100 | 0 |
| 25 μM Compound #1 | 95 | 95 | 0.093 | 83 | 87 | 13 |
| 25 μM Compound #2 | 98 | 98 | 0.108 | 96 | 98 | 2 |
| 25 μM Compound #3 | 111 | 111 | 0.111 | 99 | 89 | 11 |

The above table indicates that compound #1 compares favorably via its effect directly at melanin content level while compound #3 exhibits its effect more via cell number. Effect of compound #2 (compound outside the scope of the invention) however is comparatively insignificant.

Example 2: Effect compound #1 at different doses

Compound #1 was further examined for its effect at various doses on another fresh batch of human primary melanocytes and compared to a well-known skin lightening agent, Kojic acid.

Table 2: Cellular Melanin Content Data

| Material Tested | Calcein Fluorescence (arbitrary units) | % Viability | $OD_{405nm}$ (Melanin) | % Melanin | 100* (% Melanin/ %Viability) | % reduction in Melanin |
|---|---|---|---|---|---|---|
| 0.25% v/v DMSO control | 100 | 100 | 0.175 | 100 | 100 | 0 |
| 25 μM Kojic acid | 102 | 102 | 0.177 | 101 | 99.3 | 0.7 |
| 250 μM Kojic acid | 96 | 96 | 0.152 | 87 | 90.4 | 9.6 |
| 500 μM Kojic acid | 96 | 96 | 0.139 | 80 | 83.3 | 16.7 |
| 3.125 μM Compound #1 | 94 | 94 | 0.168 | 96 | 102.1 | Nil |

(continued)

| Material Tested | Calcein Fluorescence (arbitrary units) | % Viability | $OD_{405nm}$ (Melanin) | % Melanin | 100* (% Melanin/ %Viability) | % reduction in Melanin |
|---|---|---|---|---|---|---|
| 6.25 μM Compound #1 | 96.6 | 96.6 | 0.146 | 83 | 85.9 | 14.1 |
| 12.5 μM Compound #1 | 93.4 | 93.4 | 0.134 | 77 | 82.4 | 17.6 |
| 25 μM Compound #1 | 93.7 | 93.7 | 0.122 | 70 | 74.7 | 25.3 |
| 50 μM Compound #1 | 88.3 | 88.3 | 0.103 | 59 | 66.8 | 33.2 |

The data in Table 2 clearly indicates superiority of compound #1 over Kojic acid as compound #1 is more efficacious at concentrations much lower than Kojic acid.

## Claims

1. Use of a compound of formula (I) or a salt thereof, for reducing cellular melanin content of human skin, where,

    (i) R is:

    (a) - $(CH_2)_n$ - A, where n = 1 to 6 and A is -OH or -NH2; or
    (b) - O-$(CH_2)_n$ - X, where n = 1 to 5 and X is a halogen;

    (ii) $R_2$ is -H, alkyl, n-hexyl, aryl, alkoxy or aryloxy group; and,
    (iii) $R_3$ is -H, alkyl, aryl, alkoxy or aryloxy group,

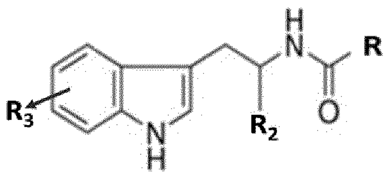

(I)

wherein said use is for cosmetic purpose.

2. Use according to claim 1 wherein, where said R is - O-$(CH_2)_n$ - X, said n = 2 and said X is chlorine, bromine or fluorine.

3. Use according to claim 1 wherein, where said R is -$(CH_2)_n$ - A, said n = 1 and said A is -OH or -$NH_2$.

4. Use according to any of claims 1 to 3 wherein said $R_2$ is an alkyl group.

5. Use according to any of claims 1 to 4 wherein $R_3$ is -H.

6. Use according to any of claims 1 to 5 wherein the compound is 2-chloroethyl [2-(1*H*-indol-3-yl)-1-methylethyl] carbamate.

7. Use according to any of claims 1 to 5 wherein the compound is 2-amino-N-[2-(1H-indol-3-yl)-1-methylethyl] acetamide acetate.

8. Use according to any of claims 1 to 7 wherein the salt of the compound is an acetate.

9. Use according to any of claims 1 to 8 wherein said melanin is contained within human primary melanocytes.

10. A method of reducing cellular melanin content of human skin comprising a step of applying thereto a compound of the formula (I) or a salt thereof, as claimed in claim 1 wherein said method is a cosmetic method.

**Patentansprüche**

1. Verwendung einer Verbindung der Formel (I) oder eines Salzes davon zur Verringerung des zellulären Melaningehalts der menschlichen Haut, wobei

   (i) R:

      (a) $-(CH_2)_n-A$, worin n = 1 bis 6 und A -OH oder -NH2 ist; oder
      (b) $-O-(CH_2)_n-X$, worin n = 1 bis 5 und X ein Halogen ist; ist,

   (ii) $R_2$ -H, Alkyl-, n-Hexyl-, Aryl-, Alkoxy- oder Aryloxygruppe ist; und
   (iii) $R_3$ -H, Alkyl-, Aryl-, Alkoxy- oder Aryloxygruppe ist,

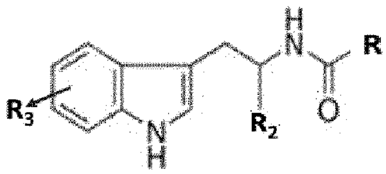

(I)

wobei die Verwendung für kosmetische Zwecke ist.

2. Verwendung nach Anspruch 1, wobei das R $-O-(CH_2)_n-X$ ist, das n = 2 und das X Chlor, Brom oder Fluor ist.

3. Verwendung nach Anspruch 1, wobei das R $-(CH_2)_n-A$ ist, das n = 1 und das A -OH oder $-NH_2$ ist.

4. Verwendung nach irgendeinem der Ansprüche 1 bis 3, wobei das $R_2$ eine Alkylgruppe ist.

5. Verwendung nach irgendeinem der Ansprüche 1 bis 4, wobei $R_3$ -H ist.

6. Verwendung nach irgendeinem der Ansprüche 1 bis 5, wobei die Verbindung 2-Chlorethyl-[2-(1H-indol-3-yl)-1-methylethyl]carbamat ist.

7. Verwendung nach irgendeinem der Ansprüche 1 bis 5, wobei die Verbindung 2-Amino-N-[2-(1H-indol-3-yl)-1-methylethyl]acetamidacetat ist.

8. Verwendung nach irgendeinem der Ansprüche 1 bis 7, wobei das Salz der Verbindung ein Acetat ist.

9. Verwendung nach irgendeinem der Ansprüche 1 bis 8, wobei das Melanin in menschlichen primären Melanozyten enthalten ist.

10. Verfahren zur Verringerung des zellulären Melaningehalts der menschlichen Haut, umfassend einen Schritt des Aufbringens einer Verbindung der Formel (I) oder eines Salzes davon, wie in Anspruch 1 beansprucht, darauf, wobei das Verfahren ein kosmetisches Verfahren ist.

## Revendications

1. Utilisation d'un composé de formule (I) ou d'un sel de celui-ci, pour réduire la teneur en mélanine cellulaire de peau humaine, où,

   (i) R est :

   (a) $-(CH_2)_n-A$, où n = 1 à 6 et A est -OH ou -NH2 ; ou
   (b) $-O-(CH_2)_n-X$, où n = 1 à 5 et X est un halogène ;

   (ii) $R_2$ est -H, un groupe alkyle, n-hexyle, aryle, alcoxy ou aryloxy ; et,
   (iii) $R_3$ est -H, un groupe alkyle, aryle, alcoxy ou aryloxy,

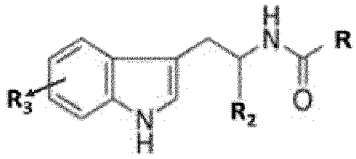

(I)

dans laquelle ladite utilisation est à destination cosmétique.

2. Utilisation selon la revendication 1, dans laquelle, lorsque ledit R est $-O-(CH_2)_n-X$, ledit n = 2 et ledit X est le chlore, brome ou fluor.

3. Utilisation selon la revendication 1, dans laquelle, lorsque ledit R est $-(CH_2)_n-A$, ledit n = 1 et ledit A est -OH ou $-NH_2$.

4. Utilisation selon l'une quelconque des revendications 1 à 3, dans laquelle ledit $R_2$ est un groupe alkyle.

5. Utilisation selon l'une quelconque des revendications 1 à 4, dans laquelle $R_3$ est -H.

6. Utilisation selon l'une quelconque des revendications 1 à 5, dans laquelle le composé est le [2-(1*H*-findol-3-yl)-1-méthyléthyl]-carbamate de 2-chloroéthyle.

7. Utilisation selon l'une quelconque des revendications 1 à 5, dans laquelle le composé est l'acétate de 2-amino-N-[2-(1*H*-indol-3-yl)-1-méthyléthyl]acétamide.

8. Utilisation selon l'une quelconque des revendications 1 à 7, dans laquelle le sel du composé est un acétate.

9. Utilisation selon l'une quelconque des revendications 1 à 8, dans laquelle ladite mélanine est contenue dans des mélanocytes primaires humains.

10. Procédé de réduction de teneur en mélanine cellulaire de peau humaine comprenant une étape d'application à celle-ci d'un composé de la formule (I) ou d'un sel de celui-ci, selon la revendication 1 dans lequel ledit procédé est un procédé cosmétique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 1903203
- US 20060135618 A, Jean
- US 20020044914 A, Dooley
- JP 56032460 A, Nihon Tokushu Noyaku Seizo
- EP 2414701 A1, Ajinonoto
- WO 9806695 A1, Sederma
- EP 0820767 A2, L'Oreal
- EP 2196190 A1, Cognis
- EP 2412701 A


### Non-patent literature cited in the description


- **THANIGAIMALAI et al.** *Bioorganic & Medicinal Chem. Letters,* 2011, vol. 21, 6824-6828
- **LIU et al.** *Chem. Res. Chinese Universities,* 2010, vol. 26 (6), 929-932
- **BAUER, K. et al.** Common Fragrance and Flavor Materials. VCH Publishers, 1990
- The CTFA Cosmetic Ingredient Handbook. 1992